# EUROPEAN PATENT SPECIFICATION

(11) **EP 3 443 116 B1**
(45) Date of publication and mention of the grant of the patent: **28.10.2020**
(21) Application number: 17719189.7
(22) Date of filing: 07.04.2017
(51) Int. Cl.: C12Q 1/68

(54) **SUB-POPULATION DETECTION AND QUANTIZATION OF RECEPTOR-LIGAND STATES FOR CHARACTERIZING INTER-CELLULAR COMMUNICATION AND INTRATUMORAL HETEROGENEITY**
UNTERPOPULATIONSDETEKTION UND -QUANTISIERUNG VON REZEPTOR-LIGAND-ZUSTÄNDEN ZUR CHARAKTERISIERUNG VON INTERZELLULÄRER KOMMUNIKATION UND INTRATUMORALER HETEROGENITÄT
DÉTECTION DE SOUS-POPULATION ET QUANTIFICATION D'ÉTATS RÉCEPTEUR-LIGAND DESTINÉES À LA CARACTÉRISATION DE LA COMMUNICATION INTERCELLULAIRE ET DE L'HÉTÉROGÉNÉITÉ INTRATUMORALE

(30) Priority: 15.04.2016 US 201662323288 P
(43) Date of publication of application: 20.02.2019
(73) Proprietor: Koninklijke Philips N.V., 5656 AG Eindhoven (NL)
(72) Inventor: SANTHANAM, Balaji Srinivasan, 5656 AE Eindhoven (NL); CHEUNG, Yee Him, 5656 AE Eindhoven (NL); AGRAWAL, Vartika, 5656 AE Eindhoven (NL); DE BONT, Johanna Maria, 5656 AE Eindhoven (NL); DIMITROVA, Nevenka, 5656 AE Eindhoven (NL)
(74) Representative: Beck, Christopher Andrew
(86) International application number: PCT/EP2017/058322
(87) International publication number: WO 2017/178345

(56) References cited:
- HYEJIN CHOI ET AL: "Transcriptome Analysis of Individual Stromal Cell Populations Identifies Stroma-Tumor Crosstalk in Mouse Lung Cancer Model", CELL REPORTS, vol. 10, no. 7, 1 February 2015 (2015-02-01), pages 1187-1201, XP055374032, US ISSN: 2211-1247, DOI: 10.1016/j.celrep.2015.01.040
- A. P. PATEL ET AL: "Single-cell RNA-seq highlights intratumoral heterogeneity in primary glioblastoma", SCIENCE, vol. 344, no. 6190, 20 June 2014 (2014-06-20), pages 1396-1401, XP055352661, ISSN: 0036-8075, DOI: 10.1126/science.1254257 cited in the application
- JORDAN A. RAMILOWSKI ET AL: "A draft network of ligand-receptor-mediated multicellular signalling in human", NATURE COMMUNICATIONS, vol. 6, 22 July 2015 (2015-07-22), page 7866, XP055374051, DOI: 10.1038/ncomms8866

## Description

### FIELD OF THE INVENTION

The present invention relates to a system and method for characterizing intercellular communication and heterogeneity in tumors, and more particularly a method for detecting sub-populations and receptor-ligand states for providing predictive information in relation to cancer and cancer treatment.

### BACKGROUND OF THE INVENTION

There is increasing awareness that tumors may be highly heterogeneous. Intratumoral heterogeneity has impeded the design, development and effective use of targeted therapies in the clinical setting. The origins of heterogeneity may be ascribed to differences in the genetic composition of the cells that constitute the tumor. Different subclones often cooperate in driving tumor growth and invasion. Thus, even directed therapies that target one or a small subset of subclones may not be effective, resulting in recurrence and metastatic disease. Differences in the genetic makeup and/or molecular composition manifest as distinct cell physiologies, some of which may be observable through imaging modalities. However, imaging captures only a birds-eye view and higher resolution at the molecular level may be essential in developing novel therapeutic strategies. Moreover, when using a sample analyzed with mere population (bulk) methods, very important molecules may not be detectable and hence achieving effective therapy planning is quite challenging. With single-cell data we strive to provide the resolution to detect these important signaling molecules in cells (and their defining cell populations) they are expressed in.

Further, cell-cell communication is an essential component in biological processes including development, lineage determination, cell differentiation and signaling. Cell communication may happen within short ranges (paracrine) or long ranges (endocrine) and in some cases, cells may signal to themselves (autocrine). Normal molecular signaling patterns among the many different cell types in order to maintain coordinated function have been studied and described at a tissue level and captured using various methods such as immunohistochemistry and other molecular assays. However, there are no methods describing how to analyze and detect signaling among cell populations in the context of diagnosis and in the context of finding therapy targets that might disrupt intratumoral signaling at the population level. The current thinking is that if there is a receptor - it may be ubiquitously expressed in a majority of cancer cells and represents a hallmark for a certain subtype of cancer.

In cancers, accumulation of genetic aberrations in a subset of cells results in alterations to "normal" roles these cells typically play in their native tissue of origin or organ. Some of these mutations may confer a proliferative growth advantage to these cells and facilitate cancer progression. As the landscape of these mutations expands, the genetic heterogeneity within the tumor increases and so does the diversity of molecular signals associated with the tumorous tissue and its environment. Such heterogeneous tumors, which are composed of multiple sub-populations pose a major challenge to therapy. This is further exacerbated under selection pressures such as chemotherapy resulting in the emergence of subclones that are resistant and may take over the tumor mass. This heterogeneity is difficult to characterize using mere population data collection strategies. The advent of single-cell technologies can be used to overcome some of these challenges to examine the extent and nature of heterogeneity.

Over the past few decades, rapid advances in next-generation sequencing technologies have had tremendous impact on both the volume and quality of genomic and molecular data. Alterations to the genome, small and big, have been implicated in several cancers. These genetic changes often result in changes at the transcript level and comprehensive estimation of transcript abundances can be obtained using RNA sequencing ("RNA-seq") using Next Generation Sequencing ("NGS") hardware (produced for example from Illumina, Life Technologies, PacBio, NanoString, etc.). These high-throughput technologies, both genome sequencing and RNA-seq, require a substantial number of cells and provide mere population averages at the overall "bulk" tumor sample (i.e., all the cells are sequenced together). Thus, these data do not reveal inherent stochasticity or systematic variations within the population. Most existing methodologies describing signaling and pathway enrichments typically utilize population data, which preclude comprehensive assessments on intrinsic heterogeneities in the tumor. More recently, the advent of single-cell sequencing has enabled information gathering at the cellular level. Specifically, single-cell RNA-seq technologies allow for sampling transcript abundance in individual cells, providing a glimpse into the underlying molecular heterogeneity and possibly a glimpse into how molecular signaling and communication is altered in tumors.

Unlike population methods, single-cell technologies offer a huge advantage since they enable information gathering at the next level of resolution while retaining cellular heterogeneity information. In heterogeneous tissues such as tumors, the transcript abundances of signaling molecules display considerable variability. Using mere population (bulk) methods, these critical molecules may not be detectable and hence achieving effective therapy planning is quite challenging. On the other hand, using single-cell data provides the resolution to detect these molecules in cells they are expressed in. Thus, signaling and pathway analyses derived from these data provide a unique perspective compared to those obtained from population data. Moreover, perhaps due to the heterogeneity, signaling mechanisms and pathways enriched in tumor cells are expected to be very different from normal molecular signaling patterns observed in different cell types that are required to maintain coordinated function. The observation that different subclones in a tumor may act in synchrony for proliferation and progression of the disease leads to the hypothesis that there is active communication between them. Such communication is mediated through a network of ligands and receptors and these interactions initiate the downstream signaling. The ligand, which is the message is communicable to all the cells in the ecosystem with a suitable receiver, the receptor. In this novel paradigm, the same cell need not synthesize the ligand and its cognate receptor for signaling to be initiated. Depending on the nature of the ligand and its stability, long-range and/or short-range communications between cells in different subclones can be established. In this invention, by leveraging the advantages of single-cell RNA-seq data, a system and a method to identify curated pairs of ligands and their cognate receptors that display checkered expression patterns within a population of cells from a tumor is disclosed.

Although single-cell technologies can be used to overcome some of the challenges in examining the extent and nature of heterogeneity, there is still a need for methods to detect and analyze signaling among cell populations in the contexts of precision diagnostics, identifying therapy targets, and precision oncology which includes individualized therapy planning for patients. Within this context, there is a need for methods for identifying sub-populations where intratumoral signaling occurs as well as identifying curated receptor-ligand pairs that may be implicated in the signaling. Hence, a framework that provides insight into intercellular communication is needed. Accordingly, a method that leverages the advantages of single-cell RNA-seq, into a quantization procedure for detecting receptor-ligand states as a measure of heterogeneity in the form of a transforming the RNA-seq data into a receptor-ligand communication map (ReLiCoMap) providing insight into intercellular communications, would be advantageous. In particular, a system for identifying cellular sub-populations and the likely receptor-ligand interactions that govern intercellular communication between the sub-populations by transforming the RNA-seq data into a receptor-ligand communication map (ReLiCoMap) would be advantageous.

Hyejin Choi et al: "Transcriptome Analysis of Individual Stromal Cell Populations Identifies Stroma-Tumor Crosstalk in Mouse Lung Cancer Model", Cell Reports, vol.10, no.7, 1 February 2015, pages 1187-1201, discloses a computational model that identifies global paracrine/autocrine crosstalk networks.

### SUMMARY OF THE INVENTION

In particular, it may be seen as an object of the present invention to provide a framework that solves the above-mentioned problems of the prior art to provide a system that utilizes single-cell RNA-seq. data into a quantization procedure for receptor-ligand states as a measure of heterogeneity. It is also an object of the present invention to provide a system that provides a visualized receptor-ligand display, such as a receptor-ligand map, or ReLiCoMap (as used herein) for identifying cellular sub-populations and the likely receptor-ligand interactions that govern intercellular communication between the sub-populations. It is a further object of the present invention to provide an alternative to the prior art. To better address this concern, according to the invention a system and a method are presented as defined in the claims.

In the context of the present invention, the term "curated gene list" is taken to mean the gene list reported by J.A. Ramilowski et al., "A draft network of ligand-receptor-mediated multicellular signalling in human," Nat. Commun., vol. 6, p. 7866, 2015.

In the context of the present invention, the term "curated receptor-ligand pair" is taken to mean curated list of 1894 receptor-ligand pairs reported by J.A. Ramilowski et al., "A draft network of ligand-receptor-mediated multicellular signalling in human," Nat. Commun., vol. 6, p. 7866, 2015.

In the context of the present invention, the term "invariant" is taken to mean values that show variation below quantile level of 0.75 of the overall standard deviation of the same values.

In the context of the present invention, the term "unsupervised clustering" or "unsupervised clustering method" is taken to mean clustering methods including but not limited to hierarchical clustering performed without any prior label information. *See, e.g.,* Gareth, J. et al., "An Introduction to Statistical Learning," Springer; 1st ed. 2013, Corr. 5th printing 2015 edition (August 12, 2013).

In the context of the present invention, the term "sub-population" is taken to mean, as an example, a partitioning of cells identified to indicate a sub-structure in the data using the R package NbClust. This package provides different indices for determining the optimal number of clusters in a data set and offers the best clustering scheme from different results to the user. However, multiple algorithms for unsupervised learning can be used in the present invention instead of hierarchical clustering, such as k-means or PAM. In addition to NbClust, there are other methods that can be used for determining the number of clusters such as the elbow, the silhouette and gap statistic method.

In the context of the present invention, the term "sub-clusters" is taken to mean a high confidence smaller cluster comprising a larger cluster. Confidence is estimated using resampling methods such as, for example, multiscale bootstrap (R package pvclust).

In the context of the present invention, the term "checkered expression" is taken to mean values that are not constant or nearly constant across all observations.

In the context of the present invention, the term "normalized expression" is taken to mean transcript abundances that are scaled for total reads from the sequencing experiment and scaled for length of the transcript. This permits comparison of transcript abundances between genes and across experiments.

In the context of the present invention, the term "ReLiCoMap" refers to a receptor-ligand communication visualization tool that displays the results obtained in a form that allows a physician or clinician to identify the receptor-ligand pairs likely to be involved in cellular communication and to devise appropriate therapies.

### BRIEF DESCRIPTION OF THE DRAWINGS

The methods according to the invention will now be described in more detail with regard to the accompanying figures. The figures showing ways of implementing the present invention and are not to be construed as being limiting to other possible embodiments falling within the scope of the attached claims.
FIG. 1 shows a pathway of steps to quantize receptor-ligand states and identify discriminative receptor-ligand pairs;
FIG. 2 shows a heatmap of receptor-ligand quantized states according to FIG. 1, in patient 5;
FIG. 3 shows a pathway of steps to identify sub-populations and identify receptor-ligand pairs that may govern intercellular communications;
FIG. 4 shows a circos (ReLiCoMap) plot depicting sub-population interactions discovered in patient 5; and
FIG. 5 is a diagram of an embodiment of a system according to the within invention.

### DETAILED DESCRIPTION OF THE INVENTION

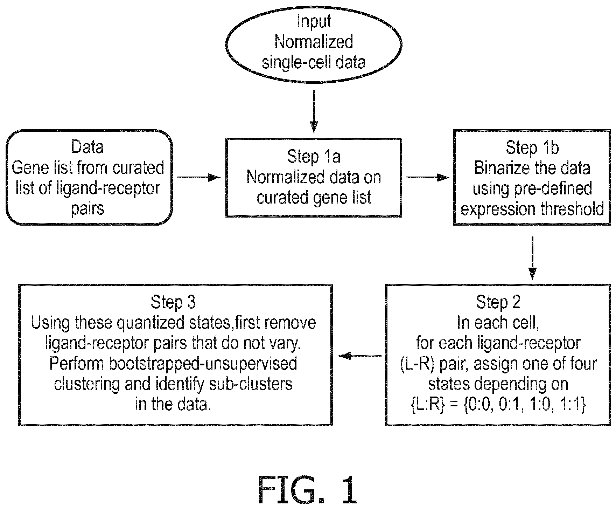

The present invention provides a system and methods for quantization of receptor-ligand states to identify components of intercellular communication and a method for sub-population detection and identification of likely receptor-ligands that orchestrate intercellular communication between sub-populations. The present invention is described in further detail below with reference made to FIGS. 1-5.

According to an embodiment of the present invention, a first process of quantization of receptor-ligand states to identify components of intercellular communication is set forth by the steps outlined in FIG. 1. Initially, single-cell RNA-seq data is generated from NGS hardware that may be employed in a hospital, service laboratory or other diagnostic facility. Typically, the NGS hardware contains computer memory and processing capabilities. Alternatively, single-cell RNA-seq data obtained from alternate sources, such as published literature, may be used. The first step, gene selection, is accomplished by identification of the receptor-ligand pairs and for each gene included, extracting their normalized transcript abundance data (Step 1a), according to the expression: ***e**_{g}* = {*e_{c}*}*_{c=1}*, _{..., *C*} and *g* ={*l_{i,}rⱼ*}_{*i*=*1*, ..., *I; j*=*1,* ..., *J*}, where *lᵢ* and *rⱼ* denote the collection of ligands and receptors respectively. ***e**_{g}* denotes a collection of expression values for genes in set {*g*} from cells {*c*}. All expression values in ***e**_{g}* smaller than a chosen threshold ε*ₜₕ* are made 0 and the rest are set to 1 to obtain ***b**_{g}. **b**_{g}* denotes the binarized expression vector of the genes in set {g}.

As an illustration of this embodiment, we utilized single-cell RNA-seq data obtained from five glioblastoma multiforme (GBM) patients, published by A.P. Patel et al., Single-cell RNA-seq highlights intratumoral heterogeneity in primary glioblastoma," Science, vol. 344, no. 6190, pp. 1396-1401, Jun. 2014. We downloaded raw data, mapped and quantified normalized transcript abundance in 430 cells from five patients. Each patient sample included 70 to 118 cells. Receptor-ligand pairs were selected from a curated list of 2557 receptor-ligand pairs reported by J.A. Ramilowski *et al.* For expression thresholding, a threshold of 16 FPKM units was selected to binarize the data (Step 1b). It should be noted that this threshold is arbitrary but is best chosen keeping in mind constraints imposed by molecular biology on detectability of transcript abundance.

The second step, Step 2 of FIG. 1, is that of quantization of receptor-ligand pairs. In every cell, each ligand *lᵢ* and receptor *rⱼ* that form a curated receptor-ligand pair *pᵢⱼ* are assigned one of four states depending on if *bₖ* = {0,1}, *k* = {*lᵢ*, *rⱼ*} and the resulting matrix of receptor-ligand states is ***S.*** In Step 3, the receptor-ligand pairs are clustered and sub-groups are extracted. Receptor-ligand pairs whose quantized states have variance 0 (*i.e.,* are invariant) are removed from matrix ***S.*** Using multiscale bootstrapped unsupervised clustering followed by identification of sub-clusters in the data, the most discriminative receptor-ligand pairs are identified. These sub-clusters may be identified by any robust cluster identification algorithm or other metrics, including selecting receptor-ligand pairs with the most varying quantized states and hierarchical clustering with dynamic tree-cutting.

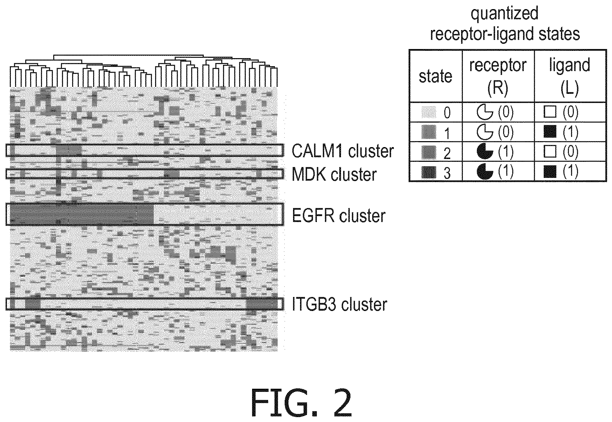

In this illustration of this embodiment, each curated receptor-ligand pair in each cell was classified into one of four states (0,1,2,3), depending on whether ligand and receptor expression was 0 or 1. For the purposes of clustering, receptor-ligand pairs quantized at states 1 or 2 (*i.e.,* either of the ligand's or receptor's expression was 0 but not both) were considered to be equidistant from the other two states (*i.e.,* 0 and 3). From this, a quantized-state matrix for receptor-ligand pairs in 430 cells is obtained. We use a heatmap along with hierarchical clustering to visualize this data for each patient. In the heatmap for patient 5 (*see* FIG. 2.), each row corresponds to a receptor-ligand pair and each column corresponds to a cell whose transcriptome has been sequenced and quantified. The values correspond to the quantization states of the receptor-ligand pairs in each cell. This approach results in the identification of prominent clusters in which the same receptor (or ligand) paired with multiple ligand (or receptor) partners cluster together. Some of the prominent clusters include *EGFR* and *ITGB3* as the participating receptors and *MDK* and *CALM1* as the participating ligands. The data show that not all cells express both ligand and receptor (state 3), and in fact a majority of them express neither (state 0). Despite this, there is evidence for checkered expression of ligands and receptors as evidenced from the fact that distinct cell populations express either the ligand or the receptor. For example, in the *EGFR* cluster, there are two broad clusters of cells-one in which cells are categorized as state 2 (or state 3) and the other in which most cells seem to be categorized as state 0 or state 1 suggesting that in these two cellular populations, the former express the receptor while the latter only express the ligands. These results point to potential cooperation between these cellular populations despite the fact that one group appears to act as the "sender" and the other the "receiver." We found that the implicated receptor-ligand pairs varied between the five patients suggesting specific genetic determinants may drive disease progression in these patients. More importantly, this also potentially highlights the inherent need and power of personalized precision medicine since each patient may require distinct therapeutic strategies.

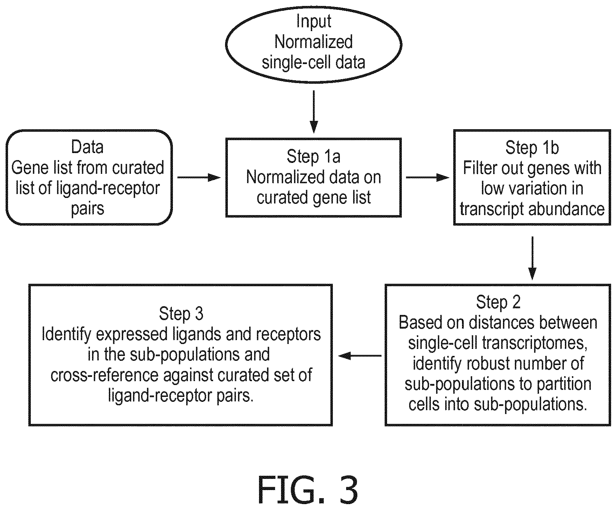

The steps of a further embodiment of the within invention are outlined in FIG. 3. In this embodiment, sub-clusters are estimated to identify components of intercellular communications. Referring to FIG. 3, Step 1 comprises gene selection. Single-cell RNA-seq. data is generated and normalized as in the first embodiment. *See* Step 1a. Alternatively, normalized data can be selected from the curate gene list of receptor-ligand pairs previously referenced. *See* Ramilowski *et al.* After removing genes that exhibit low variation in transcript abundance across the cells (Step 1b), the genes are selected and their normalized transcript abundance stored as ***e**_{g}* = {e_{c}}_{*c*=*1*, ..., *C*} and *g* ={*lᵢ*,*rj*}_{*i*=*1* ,..., *I; j*=1 ,...,} *_{J},* where *lᵢ* and *rⱼ* denote the collection of ligands and receptors respectively. ***e**_{g}* denotes a collection of expression values for genes in set {*g*} from cells {*c*}. In illustration of this embodiment, we used the gene expression matrix retained in the previous embodiment, and excluded genes that did not vary. In each patient, we considered genes whose standard deviation in transcript abundance was above the 95^{th} quantile in overall standard deviation.

In Step 2 of this embodiment, FIG. 3, sub-populations are identified using pair-wise distances between individual cells based on *e_{c}* from cells *c* = *1,* ..., *C*. Robust sub-populations are identified based on similarity between the cellular transcriptomes. The distance between two transcriptomes is defined as D = 1-SC, where SC is the Spearman's correlation coefficient between the transcriptomes and is a measure of similarity between them. Partitioning into sub-populations is permitted only if a minimum number of cells in the total population support each partition. Using the reduced set of genes from Step 1, we identified robust sub-populations in the data. We used the R package NbClust and used 30 different indices to arrive at a consensus number of partitions that identify the sub-populations. See M. Charrad et al., "NbClust: An R Package for Determining the Relevant Number of Clusters in a Data Set," J. Stat. Softw., vol. 61, no. 6, Oct. 2014. We found that the number of partitions varied between the different patients and within a patient in successive iterations. In order to robustly identify putative receptor-ligand pairs involved in intercellular communication, we repeated this procedure 500 times while limiting the number of partitions each time to utmost 6. We found that for these data, the median partitions were fairly consistent (Table 1)

**Table 1: Sub-population identification from single-cell data in five patient samples**

| Patient | Number of cells sequenced | Number of sub-populations identified (median of 500 iterations) |
|---|---|---|
| 1 (MGH26) | 118 | 3 |
| 2 (MGH28) | 94 | 3 |
| 3 (MGH29) | 75 | 4 |
| 4 (MGH30) | 73 | 3 |
| 5 (MGH31) | 70 | 3 |

Step 3 of this embodiment entails clustering and extracting of sub-groups. This step comprises identification of expressed ligands and receptors across the sub-populations and cross-referencing them against the curated set of receptor-ligand pairs. A gene (ligand or receptor) is considered to be "ON" (*i.e.,* expressed) in a sub-population of cells if its transcript abundance is more than a chosen threshold **ε***ₜₕ*, in a majority of cells comprising that partition (same threshold as described in the previous embodiment). Genes that are considered "ON" are given a value 1 and others, 0. From the curated set of receptor-ligand pairs, ligands and their cognate receptors that are likely communicating between the sub-populations are identified.

Illustrating Step 3 in this embodiment, once the cells were partitioned into sub-populations, we used binarized expression data to identify ligands and receptors that were expressed in the different sub-populations. We considered a gene (ligand or receptor) to be "ON" if their transcript abundance is above a chosen threshold (16 FPKM units), in at least a majority of the cells that make up the sub-population. We matched each potential receptor-ligand pair that is "ON" across different sub-populations with the curated set of receptor-ligand pairs (Ramilowski *et al*.) to identify the putative receptor-ligand pair(s) that underlie intercellular communication between the sub-populations identified (subset shown in Table 2 for one iteration of the procedure). This cross-referencing helps reduce potential false-positives but at the same time may limit the identification of novel, previously uncharacterized receptor-ligand pairs. It should be noted that there are clear common elements discovered (*e.g., EGFR, MDK*) that are common to both embodiments described here. We can now cross-reference against a list of drugs that target these molecules and present such a list to the physician who can then determine and ultimately decide on their suitability. For example, well known *EGFR* inhibitors including lapatinib (typically used for breast cancer) and erlotinib (typically used for some lung cancers) are FDA approved drugs for other cancers, not GBM as is the case in our illustration. However, providing this information and information on other relevant drugs that are in different stages of clinical trials, the physician presents an important data point for potential treatment.

Table 2, below, shows a communication map of possible receptor-ligand pairs in an individual patient. This kind of a communication map could represent a communication signature for the tumor of an individual patient to be used both for diagnostic and therapy planning purposes.

**Table 2: Putative intercellular communications between different sub-populations identified in patient 5. Sub-populations with both Receptor and Ligand "ON" are highlighted in red. We also provide the identity of cells in each sub-population (data not shown)**

| Receptor | Sub-population(s) with Receptor "ON" | Ligand | Sub-population(s) with Ligand "ON" |
|---|---|---|---|
| *EGFR* | Sub-populations 2, 3, 4, **5** | *HSP90AA1* | Sub-populations **5** |
| *EGFR* | Sub-populations **2,** 3, 4, 5 | *SPINK1* | Sub-population **2** |
| *ITGB3* | Sub-population **2** | *ICAM4* | Sub-populations **2** |
| *ITGB3* | Sub-population 2 | *TGM2* | Sub-population 5 |
| *GPC2* | Sub-population 2 | *MDK* | Sub-population 5 |
| *CALCRL* | Sub-populations 2, 4 | *ADM* | Sub-population 3 |
| *GPR182* | Sub-population 5 | *ADM* | Sub-population 3 |
| *PTPRS* | Sub-population **2** | *PTN* | Sub-population **2** |
| *SLC16A4* | Sub-population 5 | *HLA-E* | Sub-population 2 |

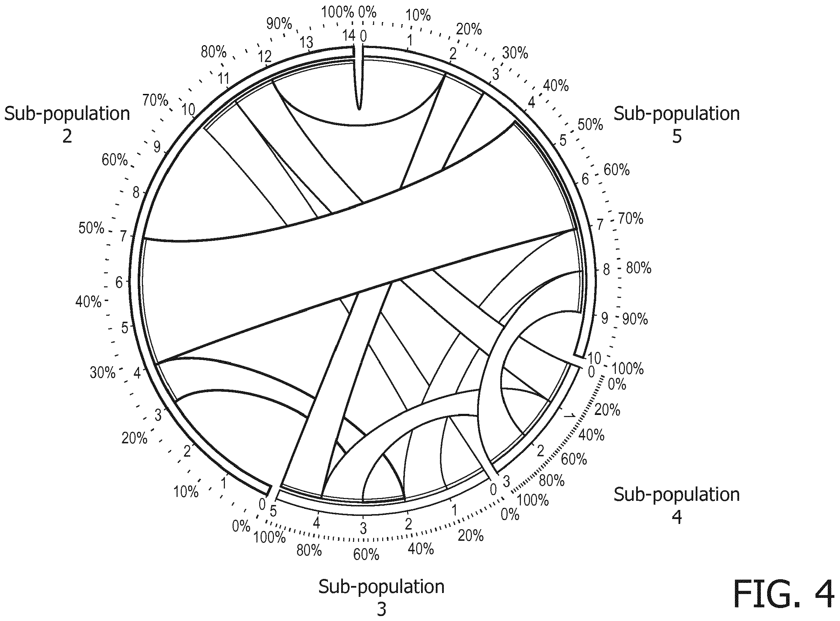

We visualize this communication map in FIG. 4 using a ReLiCoMap circular "circos" plot. This visualization tool can be used to identify the sub-population(s) that may act as communication hubs, i.e., those that appear to be interacting with many of the other sub-populations. In this particular example visualized on our results for patient 5, we find that sub-population 1 does not interact with any of the other sub-populations (hence absent from our visualization). Sub-populations 2 and 5 in our results seem to share significant number interactions (width of the ribbons connecting them). These interactions include those in which the receptor is in the "ON" state in sub-population 2 while the ligand is "ON" in sub-population 5 (5 interactions; green ribbon) and interactions in which the receptor is "ON" in sub-population 5 while the ligand is "ON" in sub-population 2 (2 interactions; magenta ribbon). It should be noted that this resolution is unavailable if mere population data collection strategies are used. The within method leverages the advantages of single-cell data to identify these patterns of signaling and visualizes them in a way that provides actionable information to clinicians.

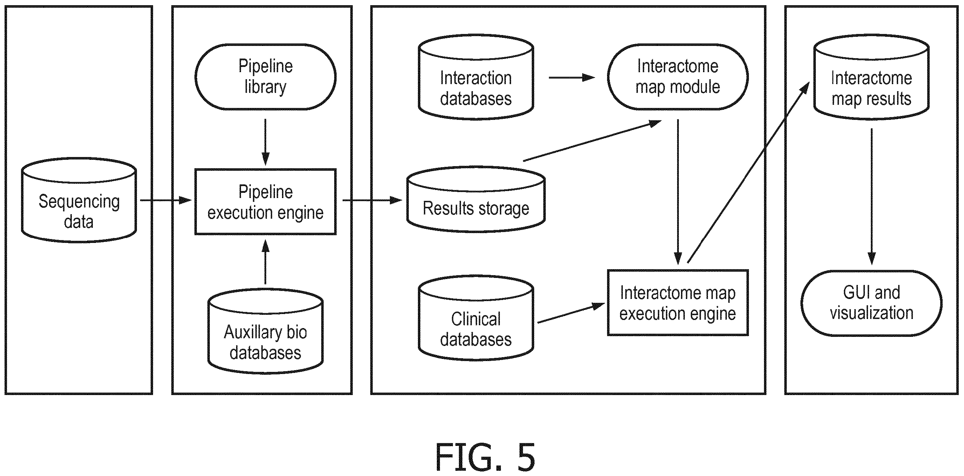

FIG. 5 outlines an end-to-end system that provides an interface to the RNA sequencing machine (or other system) to obtain sequencing data, copy the data from the sequencer to a storage device, process this data to quantify normalized transcript abundance and to identify critical components that are likely involved in intercellular communication. This discovery is visualized in a way that provides critical clinical utility to the physician and inform his/her therapy planning, monitoring and diagnostic decisions.

In yet another aspect of the invention, single-cell RNA-seq. is obtained from the sequencing machine (or any other source) as fastq files (or other suitable format). The pipeline execution engine is run to map the sequencing data by aligning the short reads to a reference sequence such as a reference genome or by stitching together a large number of these short read sequences to form a longer contiguous region. Auxiliary Bio databases provide these pieces of information including but not limited to the reference genome sequence, gene models and their coordinates in the genome. The results from these processing steps are stored in the Results storage database and can be accessed by the user at any time. The Interactome Map Module provides methodologies, such as a ReLiCoMap, to discover potential interactions from the sequencing data. A reference database like the Interactions database provides a cross-referenceable system that limits the scope of interactions to those that have been curated. The Interactome Map Execution Engine constructs the interactome map results which are stored. These results are cross-referenced with the Clinical database module to identify any actionable clinical information that may be of benefit to a clinician. This information may include a list of FDA approved drugs or those in clinical trials that are known to target components identified by the Interactome Map Execution Engine. The results are then visualized through a graphical user interface.

## Claims

1. A system for identifying receptor-ligand pairs active in intercellular communications relating to cell replication by transforming single cell RNA-seq. data into a visualized display, wherein the system is for cellular sub-population detection and identification of receptor-ligands that orchestrate intercellular communication between the sub-populations, the system comprising:
a sequencer for providing RNA-seq data from a plurality of individual cells of a cancer or tumor;
an interface in communication with the sequencer for receiving the RNA-seq data from the sequencer, said interface configured to perform the steps of;
obtaining and storing a plurality of normalized single-cell data sets generated by the sequencer;
obtaining a plurality of data sets from a curated gene list of receptor-ligand pairs;
normalizing the data from the curated gene list;
inputting reference genome sequences from library and auxiliary bio data bases;
selecting genes into a gene set by extracting their normalized transcript abundance data, assigning states to each receptor-ligand pair in each cell, removing genes that exhibit low variation in transcript abundance from the set, applying unsupervised clustering to identify sub-clusters and providing sub-cluster data; wherein the sub-clusters comprise cellular sub-populations identified based on pair-wise differences between individual cells and similarity of cellular transcriptomes; and
cross-referencing the selected gene data with the reference genome sequences data and the normalized curated list of receptor-ligand pairs, thereby identifying expressed ligands and receptors across the cellular sub-populations, and storing the results in a results storage database;
an output device, in communication with the storage database through an interface, wherein said output device is configured to obtain the identified sub-cluster and receptor-ligand data from the storage device, and to provide a display in the form of a heatmap or ReLiCoMap from which a clinician can identify receptor-ligand pairs that orchestrate intercellular communication between the identified sub-populations within the cancer or tumor.

2. A method for quantization of receptor-ligand states in genes to identify components of intercellular communication, wherein the method is for cellular sub-population detection and identification of receptor-ligands that orchestrate intercellular communication between the sub-populations, the method comprising the steps of:
obtaining a plurality of normalized single-cell data sets generated by RNA-seq of a plurality of single cells from a tumor;
obtaining a plurality of data sets from a curated gene list of receptor-ligand pairs;
normalizing the data from the curated gene list;
selecting genes by extracting their normalized transcript abundance data;
assigning states to each curated receptor-ligand pair in each cell depending on binarized levels of ligand and receptor, thereby forming a matrix of receptor-ligand states;
extracting sub-groups from the matrix that are invariant;
applying unsupervised clustering to identifying sub-clusters, wherein the sub-clusters comprise cellular sub-populations identified based on pair-wise differences between individual cells and similarity of cellular transcriptomes; and
identifying expressed ligands and receptors across the cellular sub-populations and cross-referencing against the curated set of receptor-ligand pairs, thereby identifying receptor-ligand pairs that orchestrate intercellular communication between the identified sub-populations.

3. A method for estimation of sub-clusters in genes to identify components of intercellular communication, wherein the method is for cellular sub-population detection and identification of receptor-ligands that orchestrate intercellular communication between the sub-populations, the method comprising the steps of:
obtaining a plurality of normalized single-cell data sets generated by RNA-seq. of a plurality of single cells from a tumor;
obtaining a plurality of data sets from a curated list of receptor-ligand pairs, including;
normalizing the data from the curated gene list;
selecting genes into a gene set by extracting their normalized transcript abundance data, and removing genes that exhibit low variation in transcript abundance from the set;
identifying cellular sub-populations within the set based on pair-wise differences between individual cells and similarity of cellular transcriptomes; and
identifying expressed ligands and receptors across the cellular sub-populations and cross-referencing against the curated set of receptor-ligand pairs, thereby identifying receptor-ligand pairs that orchestrate intercellular communication between the identified sub-populations.

## Patentansprüche

1. System zur Identifizierung von Rezeptor-Ligand-Paaren, die in der interzellulären Kommunikation im Zusammenhang mit der Zellreplikation aktiv sind, durch Transformation der Einzelzell-RNA-Seq. Daten in eine visualisierte Anzeige, wobei das System zur Detektion von zellulären Subpopulationen und zur Identifizierung von Rezeptor-Liganden dient, die die interzelluläre Kommunikation zwischen den Subpopulationen orchestrieren, wobei das System umfasst:
einen Sequenzer zum Bereitstellen von RNA-seq-Daten aus einer Vielzahl einzelner Zellen eines Krebses oder Tumors;
eine Schnittstelle in Kommunikation mit dem Sequenzer zum Empfangen der RNA-Seq-Daten vom Sequenzer, wobei die Schnittstelle konfiguriert ist, um die folgenden Schritte auszuführen:
Erhalten und Speichern einer Vielzahl von normalisierten Einzelzelldatensätzen, die vom Sequenzer erzeugt werden;
Erhalten einer Vielzahl von Datensätzen aus einer kuratierten Genliste von Rezeptor-Ligand-Paaren;
Normalisierung der Daten aus der kuratierten Genliste;
Eingabe von Referenzgenomsequenzen aus Bibliotheks- und Hilfsbiodatenbanken;
Auswählen von Genen in einen Gensatz durch Extrahieren ihrer normalisierten Transkriptionshäufigkeitsdaten, Zuweisen von Zuständen zu jedem Rezeptor-Ligand-Paar in jeder Zelle, Entfernen von Genen, die eine geringe Variation der Transkriptionshäufigkeit aufweisen, aus dem Satz, Anwenden einer unbeaufsichtigten Clusterbildung, zur Identifizierung von Subclustern und Bereitstellung von Subclusterdaten; wobei die Subcluster zelluläre Subpopulationen umfassen, die basierend auf paarweisen Unterschieden zwischen einzelnen Zellen und Ähnlichkeit von zellulären Transkriptomen identifiziert wurden; und
Querverweisen der ausgewählten Gendaten mit den Referenzgenomsequenzdaten und der normalisierten kuratierten Liste von Rezeptor-Ligand-Paaren, wodurch exprimierte Liganden und Rezeptoren über die zellulären Subpopulationen hinweg identifiziert werden, und die Ergebnisse in einer Ergebnisspeicherdatenbank gespeichert werden;
ein Ausgabegerät in Kommunikation mit der Speicherdatenbank über eine Schnittstelle, wobei das Ausgabegerät konfiguriert ist, um die identifizierten Subcluster- und Rezeptor-Ligandendaten von dem Speichergerät zu erhalten, und eine Anzeige in Form einer Heatmap oder ReLiCoMap bereitzustellen, anhand derer ein Kliniker Rezeptor-Ligand-Paare identifizieren kann, die die interzelluläre Kommunikation zwischen den identifizierten Subpopulationen innerhalb des Krebses oder Tumors orchestrieren.

2. Verfahren zur Quantisierung von Rezeptor-Ligand-Zuständen in Genen zur Identifizierung von Komponenten der interzellulären Kommunikation, wobei das Verfahren zur Detektion der zellulären Subpopulation und zur Identifizierung von Rezeptor-Liganden dient, die die interzelluläre Kommunikation zwischen den Subpopulationen orchestrieren, wobei das Verfahren die folgenden Schritte umfasst:
Erhalten einer Vielzahl von normalisierten Einzelzelldatensätzen, die durch RNA-Sequenz einer Vielzahl von Einzelzellen aus einem Tumor erzeugt wurden;
Erhalten einer Vielzahl von Datensätzen aus einer kuratierten Genliste von Rezeptor-Ligand-Paaren;
Normalisierung der Daten aus der kuratierten Genliste;
Auswahlen von Genen durch Extrahieren ihrer normalisierten Transkriptionshäufigkeitsdaten;
Zuweisen von Zuständen zu jedem kuratierten Rezeptor-Ligand-Paar in jeder Zelle in Abhängigkeit von binärisierten Spiegeln von Ligand und Rezeptor, wodurch eine Matrix von Rezeptor-Ligand-Zuständen gebildet wird;
Extrahieren von Untergruppen aus der Matrix, die unveränderlich sind;
Anwenden von unbeaufsichtigtem Clustering zur Identifizierung von Subclustern, wobei die Subcluster zelluläre Subpopulationen umfassen, die basierend auf paarweisen Unterschieden zwischen einzelnen Zellen und Ähnlichkeit von zellulären Transkriptomen identifiziert wurden; und
Identifizieren exprimierter Liganden und Rezeptoren in den zellulären Subpopulationen und Querverweisen auf den kuratierten Satz von Rezeptor-Ligand-Paaren, wodurch Rezeptor-Ligand-Paare identifiziert werden, die die interzelluläre Kommunikation zwischen den identifizierten Subpopulationen orchestrieren.

3. Verfahren zur Abschätzung von Subclustern in Genen zur Identifizierung von Komponenten der interzellulären Kommunikation, wobei das Verfahren zur Detektion der zellulären Subpopulation und zur Identifizierung von Rezeptorliganden dient, die die interzelluläre Kommunikation zwischen den Subpopulationen orchestrieren, wobei das Verfahren die folgenden Schritte umfasst:
Erhalten einer Vielzahl von normalisierten Einzelzelldatensätzen, die durch RNA-seq. einer Vielzahl von Einzelzellen aus einem Tumor erzeugt wurden;
Erhalten einer Vielzahl von Datensätzen aus einer kuratierten Liste von Rezeptor-Ligand-Paaren, einschließlich;
Normalisierung der Daten aus der kuratierten Genliste;
Auswählen von Genen in einen Gensatz durch Extrahieren ihrer normalisierten Transkriptionshäufigkeitsdaten und Entfernen von Genen, die eine geringe Variation der Transkriptionshäufigkeit aufweisen, aus dem Satz;
Identifizieren von zellulären Subpopulationen innerhalb des Satzes basierend auf paarweisen Unterschieden zwischen einzelnen Zellen und Ähnlichkeit von zellulären Transkriptomen; und
Identifizieren exprimierter Liganden und Rezeptoren in den zellulären Subpopulationen und Querverweisen auf den kuratierten Satz von Rezeptor-Ligand-Paaren, wodurch Rezeptor-Ligand-Paare identifiziert werden, die die interzelluläre Kommunikation zwischen den identifizierten Subpopulationen orchestrieren.

## Revendications

1. Système d'identification de paires récepteur-ligand actives dans des communications intercellulaires relatives à la réplication cellulaire par transformation des données d'une séquence d'ARN à cellules uniques dans un affichage visualisé, où le système sert à une détection de sous-populations cellulaires et à une identification de récepteurs-ligands qui orchestrent une communication intercellulaire entre les sous-populations, le système comprenant:
un séquenceur pour fournir des données d'une séquence d'ARN à partir d'une pluralité de cellules individuelles d'un cancer ou d'une tumeur;
une interface en communication avec le séquenceur pour recevoir les données d'une séquence d'ARN du séquenceur, ladite interface étant configurée pour exécuter les étapes de:
obtenir et stocker une pluralité d'ensembles de données de cellules uniques normalisées générées par le séquenceur;
obtenir une pluralité d'ensembles de données à partir d'une liste de gènes organisée de paires récepteur-ligand;
normaliser les données de la liste de gènes organisée;
entrer des séquences génomiques de référence à partir de une base de données de bibliothèque et de une base de données biologiques auxiliaire;
sélectionner des gènes dans un ensemble de gènes en extrayant leurs données d'abondance de transcription normalisées, attribuer des états à chaque paire récepteur-ligand dans chaque cellule, éliminer des gènes qui présentent une faible variation d'abondance de transcription de l'ensemble, appliquer un regroupement non supervisé pour identifier des sous-grappes et fournir des données de sous-grappes; où les sous-grappes comprennent des sous-populations cellulaires identifiées sur la base de différences par paires entre des cellules individuelles et de la similitude des transcriptomes cellulaires; et
réaliser des références croisés des données génétiques sélectionnées avec les données de séquences génomiques de référence et la liste organisée normalisée de paires récepteur-ligand, identifiant ainsi des ligands et récepteurs exprimés dans les sous-populations cellulaires, et stocker les résultats dans une base de données de stockage des résultats;
un dispositif de sortie, en communication avec la base de données de stockage via une interface, où ledit dispositif de sortie est configuré pour obtenir les données des sous-grappes et des récepteurs-ligands identifiés à partir du dispositif de stockage, et pour fournir un affichage sous la forme d'une carte thermique ou ReLiCoMap à partir de laquelle un clinicien peut identifier des paires récepteur-ligand qui orchestrent une communication intercellulaire entre les sous-populations identifiées au sein du cancer ou de la tumeur.

2. Procédé de quantification des états récepteur-ligand dans des gènes pour identifier des composants de communication intercellulaire, où le procédé est pour une détection de sous-populations cellulaires et une identification de récepteurs-ligands qui orchestrent une communication intercellulaire entre les sous-populations, le procédé comprenant les étapes de:
obtenir une pluralité d'ensembles de données de cellules uniques normalisées générées par une séquence d'ARN d'une pluralité de cellules uniques à partir d'une tumeur;
obtenir une pluralité d'ensembles de données à partir d'une liste de gènes organisée de paires récepteur-ligand;
normaliser les données de la liste de gènes organisée;
sélectionner des gènes en extrayant leurs données d'abondance de transcription normalisées;
attribuer des états à chaque paire récepteur-ligand organisée dans chaque cellule en fonction des niveaux binarisés de ligand et de récepteur, formant ainsi une matrice de états de récepteur-ligand;
extraire des sous-groupes de la matrice qui sont invariants;
appliquer un regroupement non supervisé pour identifier des sous-grappes, où les sous-grappes comprennent des sous-populations cellulaires identifiées sur la base de différences par paires entre des cellules individuelles et d'une similitude des transcriptomes cellulaires; et
identifier des ligands et des récepteurs exprimés dans les sous-populations cellulaires et réaliser des références croisées par rapport à l'ensemble organisé de paires récepteur-ligand, identifiant ainsi les paires récepteur-ligand qui orchestrent une communication intercellulaire entre les sous-populations identifiées.

3. Procédé d'estimation de sous-grappes en gènes pour identifier des composants de communication intercellulaire, où le procédé est pour une détection de sous-populations cellulaires et une identification de récepteurs-ligands qui orchestrent une communication intercellulaire entre les sous-populations, le procédé comprenant les étapes de:
obtenir une pluralité d'ensembles de données de cellules uniques normalisées générées par une séquence d'ARN d'une pluralité de cellules uniques à partir d'une tumeur;
obtenir une pluralité d'ensembles de données à partir d'une liste organisée de paires récepteur-ligand, comprenant;
normaliser les données de la liste de gènes organisée;
sélectionner des gènes dans un ensemble de gènes en extrayant leurs données d'abondance de transcription normalisées, et en éliminant des gènes qui présentent une faible variation d'abondance de transcription de l'ensemble;
identifier des sous-populations cellulaires dans l'ensemble sur la base de différences par paires entre des cellules individuelles et d'une similitude de transcriptomes cellulaires; et
identifier des ligands et des récepteurs exprimés dans les sous-populations cellulaires et réaliser des références croisées par rapport à l'ensemble organisé de paires récepteur-ligand, identifiant ainsi les paires récepteur-ligand qui orchestrent une communication intercellulaire entre les sous-populations identifiées.
